# EUROPEAN PATENT APPLICATION

(11) **EP 4 279 611 A1**
(43) Date of publication of application: **22.11.2023**
(21) Application number: 22382471.5
(22) Date of filing: 17.05.2022
(51) Int. Cl.: C12Q 1/6886

(54) **IN VITRO METHOD FOR SCREENING, DIAGNOSIS AND/OR PROGNOSIS OF COLORECTAL CANCER**

(71) Applicant: Fundación Instituto de Estudios Ciencias de la Salud de Castilla y León (IECSCYL-IBSAL), 37007 Salamanca (ES); Fundación Imdea Alimentación, 28049 Madrid (ES)
(72) Inventor: PEREA GARCÍA, José, 37007 Salamanca (ES); GONZÁLEZ SARMIENTO, Rogelio, 37007 Salamanca (ES); RAMIREZ DE MOLINA, Ana, 28049 Madrid (ES); FERNÁNDEZ ÁLVAREZ, Lara P, 28049 Madrid (ES)
(74) Representative: Hoffmann Eitle

(57) **Abstract**

The present invention refers a method for differentially screening, diagnosis and/or prognosis of colorectal cancer among men and women subjects, due to the sex disparities existing between them. In a preferred embodiment, the present invention is particularly focused on screening, diagnosis and/or prognosis early-onset colorectal cancer (EOCRC) which occurs in persons with less than 50 years of age.

## Description

### FIELD OF THE INVENTION

The present invention refers to the medical field. Particularly, the present invention refers to a method for differentially screening, diagnosis and/or prognosis of colorectal cancer among men and women subjects, due to the sex disparities existing between them. In a preferred embodiment, the present invention is particularly focused on screening, diagnosis and/or prognosis early-onset colorectal cancer (EOCRC) which occurs in persons with less than 50 years of age.

### STATE OF THE ART

EOCRC is defined as colon and rectal cancers diagnosed in persons with less than 50 years of age.

EOCRC incidence rates are increasing nowadays. Although little is known about the biological mechanisms responsible of EOCRC, it has recently been observed putative disparities by sex what suggests that metabolic dysregulation characterized by obesity, nutrition and sedentary lifestyle, which also debuts with particularities among men and women, may contribute to the development and particularities of EOCRC.

The biology of EOCRC is distinctive compared to CRC in individuals older than 50 years. Young patients are often characterized by the presence of different genomic landscapes by sex. Although molecular mechanisms contributing to them remain unknown, variation in levels of sex-steroid hormones could contribute to sex-specific metabolic phenotypes of CRC. Thus, due to these intrinsic disparities, between other reasons, no specific marker for EOCRC has been found in peripheral blood to date.

Epidemiological and experimental studies support the key role of metabolic health in CRC prevention and prognosis. Moreover, it has been demonstrated that expression panels of metabolism related genes have been useful as biomarkers of cancer prognosis and metabolic health. Interestingly, in women related cancers, the evidence suggest that the metabolic health status can increase risk for endometrial and postmenopausal breast cancers, regardless of whether a woman is overweight or obese.

On the other hand, younger patients (less than 50 years of age) are often diagnosed at a later stage of the disease, when it is more challenging to treat.

So, there is an unmet medical need of finding reliable strategies for differentially screening, diagnosis and/or prognosis colorectal cancer, particularly EOCRC, among men and women subjects, with the objective of treating the patients at an early stage of the disease thus increasing the effectiveness of the therapy.

The present invention is focused on solving this problem and method for differentially screening, diagnosis and/or prognosis of colorectal cancer among men and women subjects is herein provided, which is particularly focused on screening, diagnosis and/or prognosis EOCRC which occurs in persons with less than 50 years of age.

### DESCRIPTION OF THE INVENTION

The present invention refers to a method for differentially screening, diagnosis and/or prognosis of colorectal cancer among men and women subjects. In a preferred embodiment, the present invention is particularly focused on screening, diagnosis and/or prognosis EOCRC which occurs in persons with less than 50 years of age.

Particularly, with the aim of identifying metabolic sex disparities in EOCRC, the inventors of the present invention conducted a plasma expression profile analysis of metabolic health related genes in EOCRC patients.

As shown in the results provided by the present invention (see **Example 2)** novel biomarkers of EOCRC have been identified which show intrinsic sex disparities. Particularly, according to the present invention, EOCRC is associated with an upregulation of all metabolic health related pathways in men patients and, among them, mainly *LDLR* (and also *CPT1A)* predicted CRC development with a *c-index* of 0.991. By contrast, women that developed EOCRC characteristically exhibited upregulation of IR routes and downregulation of immune responses. In women, only *PTGS2* expression was enough to predict EOCRC development (*beta*=-1.35 95%CI (-3.13, -0.463), *c-index=0.896*)*.*

On the other hand, only one gene, low-density lipoprotein receptor-related protein 1B (*LRP1B*) was upregulated in both men and women (*beta*=1.62 95%CI (0.674, 2.99), *Bonferroni p-value*=0.003) and *beta*=2.3 95%CI (0.837, 4.91), *Bonferroni p-value=0.002*) respectively).

Indeed, since *LRP1B* is upregulated in both men and women, it can be considered as the *special technical feature* conferring unity to the present invention.

So, according to the results herein provided, the first embodiment of the present invention refers to an *in vitro* method for screening, diagnosis and/or prognosis of colorectal cancer in a subject which comprises: a) assessing the level of expression of the gene *PTGS2* in a biological sample obtained from the subject when the subject is a woman, in combination with the level of expression of the gene *LRP1B*, wherein the identification of a level of expression of the gene *LRP1B* higher, and a level of expression of the gene *PTGS2* lower, than a pre-established level of expression determined in healthy subjects, is an indication that the woman subject is suffering from colorectal cancer; or b) assessing the level of expression of the gene *LDLR* in a biological sample obtained from the subject when the subject is a man, in combination with the level of expression of the gene *LRP1B*, wherein the identification of a level of expression of the genes higher than a pre-established level of expression determined in healthy subjects, is an indication that the man subject is suffering from colorectal cancer.

The second embodiment of the present invention refers to the *in vitro* use of the gene *PTGS2* in combination with the gene *LRP1B* for screening, diagnosis and/or prognosis of colorectal cancer in women, or the gene *LDLR* in combination with the gene *LRP1B* for the screening, diagnosis and/or prognosis of colorectal cancer in men.

The third embodiment of the present invention refers to the use of a kit consisting of reagents for assessing the level of expression of the gene *PTGS2* in combination with *LRP1B* in the screening, diagnosis and/or prognosis of colorectal cancer in women, or of a kit consisting of reagents for assessing the level of expression the gene *LDLR* in combination with *LRP1B* in the screening, diagnosis and/or prognosis of colorectal cancer in men.

In a preferred embodiment, the present invention comprises assessing the level of expression of the gene *LDLR* in combination with the level of expression of the genes *LRP1B* and *CPT1A* when the subject is a man.

In a preferred embodiment of the invention the subject is under 50 years of age.

In a preferred embodiment of the invention the biological sample is selected from serum, plasma or blood.

In a preferred embodiment of the invention the results are confirmed by means of an image technique, preferably colonoscopy.

The fourth embodiment of the present invention refers to a kit consisting of reagents for assessing the level of expression of the gene *PTGS2* in combination with *LRP1B* or for assessing the level of expression the gene *LDLR* in combination with *LRP1B* and optionally with *CPT1A.*

The fifth embodiment of the present invention refers to an *in vitro* method for recommending a treatment to a subject which comprises performing the above described method for screening, diagnosis and/or prognosis of colorectal cancer, wherein: a) treatment with inflammatory and immune system modulating agents is recommended to women subjects when a level of expression of the gene *PTGS2* lower, and a level of expression of the gene *LRP1B* higher, than a pre-established level of expression determined in healthy subjects, have been identified; b) treatment with lipid-lowering agents and/or agents modulating lipid metabolism such as metformin or statins is recommended to men subjects when level of expression of the genes *LDLR* and/or *CPT1A* higher and level of expression of the gene *LRP1B* also higher, than a pre-established level of expression determined in healthy subjects, have been identified; or c) treatment with lipid-lowering agents and/or agents modulating lipid metabolism such as metformin or statins is recommended to woman subjects when level of expression of the gene *LRP1B* is higher than a pre-established level of expression determined in healthy subjects, have been identified.

The present invention also refers to a) inflammatory and immune system modulating agents for use is the treatment of women subjects when a level of expression of the gene *PTGS2* lower, and a level of expression of the gene *LRP1B* higher, than a pre-established level of expression determined in healthy subjects, have been identified; b) lipid-lowering agents and/or agents modulating lipid metabolism such as metformin or statins for use in the treatment of men subjects when level of expression of the genes *LDLR* and/or *CPT1A* higher and level of expression of the gene *LRP1B* also higher, than a pre-established level of expression determined in healthy subjects, have been identified; or c) lipid-lowering agents and/or agents modulating lipid metabolism such as metformin or statins for use in the treatment of woman subjects when level of expression of the gene *LRP1B* is higher than a pre-established level of expression determined in healthy subjects, have been identified.

Alternatively, the present invention refers to a) a method for treating women subjects when a level of expression of the gene *PTGS2* lower, and a level of expression of the gene *LRP1B* higher, than a pre-established level of expression determined in healthy subjects, have been identified, which comprises administering a pharmaceutically effective dose of inflammatory and immune system modulating agents; b) a method for treating men subjects when a level of expression of the genes *LDLR* and/or *CPT1A* higher and a level of expression of the gene *LRP1B* also higher, than a pre-established level of expression determined in healthy subjects, have been identified which comprises administering a therapeutically effective dose of lipid-lowering agents and/or agents modulating lipid metabolism such as metformin or statins for use in the treatment of; or c) a method for treating woman subjects when a level of expression of the gene *LRP1B* higher than a pre-established level of expression determined in healthy subjects has been identified, which comprises administering a pharmaceutically effective dose of lipid-lowering agents and/or agents modulating lipid metabolism such as metformin or statins.

The present invention also refers to a method for detecting the level of expression of a gene in a biological sample obtained from the subject at risk of developing colorectal cancer, the method comprising: (a) contacting the test sample with a primer specific to the genes *PTGS2* and *LRP1B* when the subject is a woman, or contacting the test sample with a primer specific to the genes *LDLR* and *LRP1B* when the subject is a man; (b) amplifying to produce an amplification product in the test sample; and (c) measuring the expression level by determining the level of the amplification product in the test sample.

In a preferred embodiment, the present invention is a computer-implemented invention, wherein a processing unit (hardware) and a software are configured to: a) receive the expression values of any of the above cited biomarkers or signatures, b) process the concentration level values received for finding substantial variations or deviations, and c) provide an output through a terminal display of the variation or deviation of the concentration level, wherein the variation or deviation of the concentration level indicates that the subject may be suffering from colorectal cancer and, optionally, this result is confirmed by an image technique, preferably colonoscopy.

For the purpose of the present invention the following terms are defied:
- The expression "pre-established" level of expression refers to a threshold value obtained after assessing the level of expression in healthy subjects (i.e. subjects not suffering from colorectal cancer).
- By "comprising" it is meant including, but not limited to, whatever follows the word "comprising". Thus, use of the term "comprising" indicates that the listed elements are required or mandatory, but that other elements are optional and may or may not be present.

- By "consisting of' it is meant "including, and limited to", whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in a subject suffering from colorectal cancer. The exact amount required will vary from subject to subject, depending on the age, and general condition of the subject, the severity of the condition being treated, mode of administration, and the like.

### Description of the figures

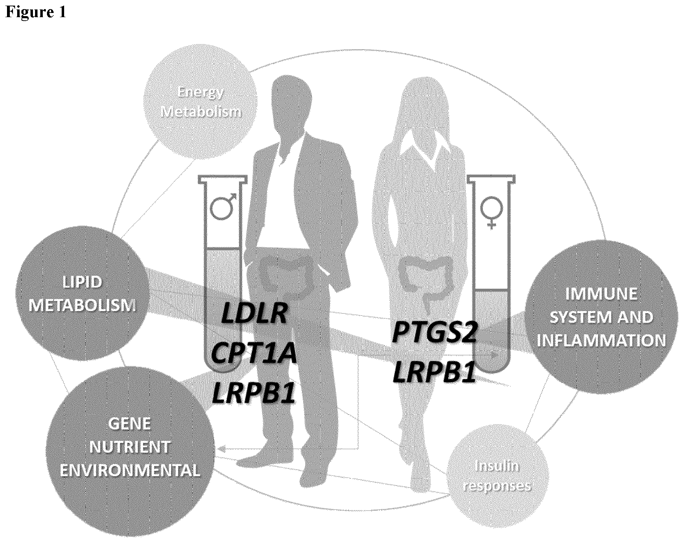

Figure 1. Sex disparities in metabolic transcriptomic features identify novel biomarkers of EOCRC.

### Detailed description of the invention

The present invention is illustrated by means of the Examples set below without the intention of limiting its scope of protection.

### Example 1. Material and methods

### Example 1.1. Patients

Fifty EOCRC patients from the Spanish Early-onset Colorectal cancer Cohort Consortium (SECOC) diagnosed with pathologically confirmed colorectal cancer (CRC) were included in the study using blood collected previously to any treatment, and 30 controls of cancer-free individuals from the Platform for Clinical Trials in Nutrition and Health (GENYAL Platform) of IMDEA-Food Institute. Main demographic features of both cases and controls populations are shown in **Table 1.**

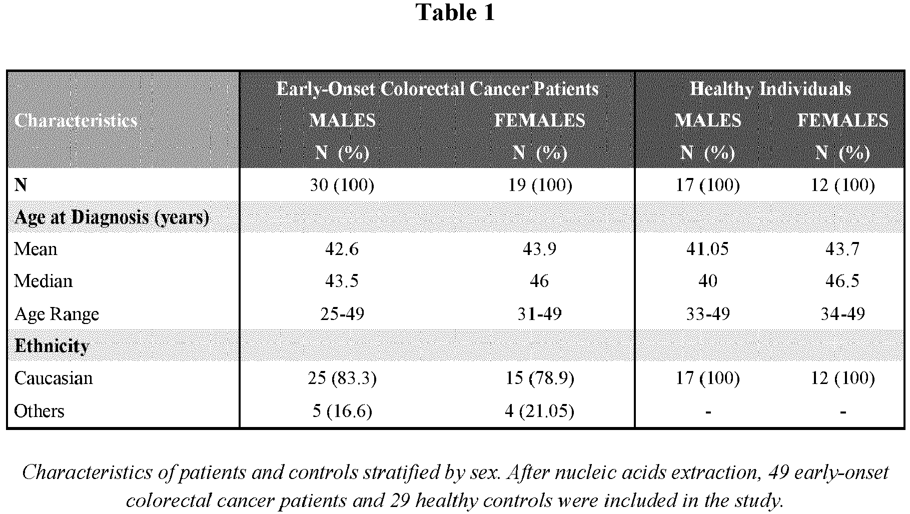

### Example 2. Results

### Example 2.1. Gene expression analysis

The inventors of the present invention analyzed peripheral blood expression of 23 genes belonging to five main metabolic health related pathways, that play an essential role in metabolic health maintenance: Lipid metabolism (LM), energetic metabolism (EM), immune system and inflammation (IS), insulin responses (IR) and gene-nutrient-environmental interactions (GNE). Then, the inventors of the present invention searched for sex-specific biomarkers of EOCRC risk.

**Table 2** shows a comparative gene expression analysis between early-onset colorectal cancer individuals and healthy controls stratified by sex. Particularly, **Table 2A** and **Table 2B** show a comparative expression Analysis of Metabolic health related genes in men and women with EOCRC respectively.

A total of seventeen genes were significantly upregulated among men with EOCRC: *ABCA1, CD36, CLOCK, CPT1A, FASN, FLCN, FNIP2, INFg, IL6, INSR, JAK1, LDLR, MTHFD, PPARg, TFAM, TNFa* and *LRP1B* (see **Table 2A).** Globally, these genes belong to all analyzed metabolic pathways.

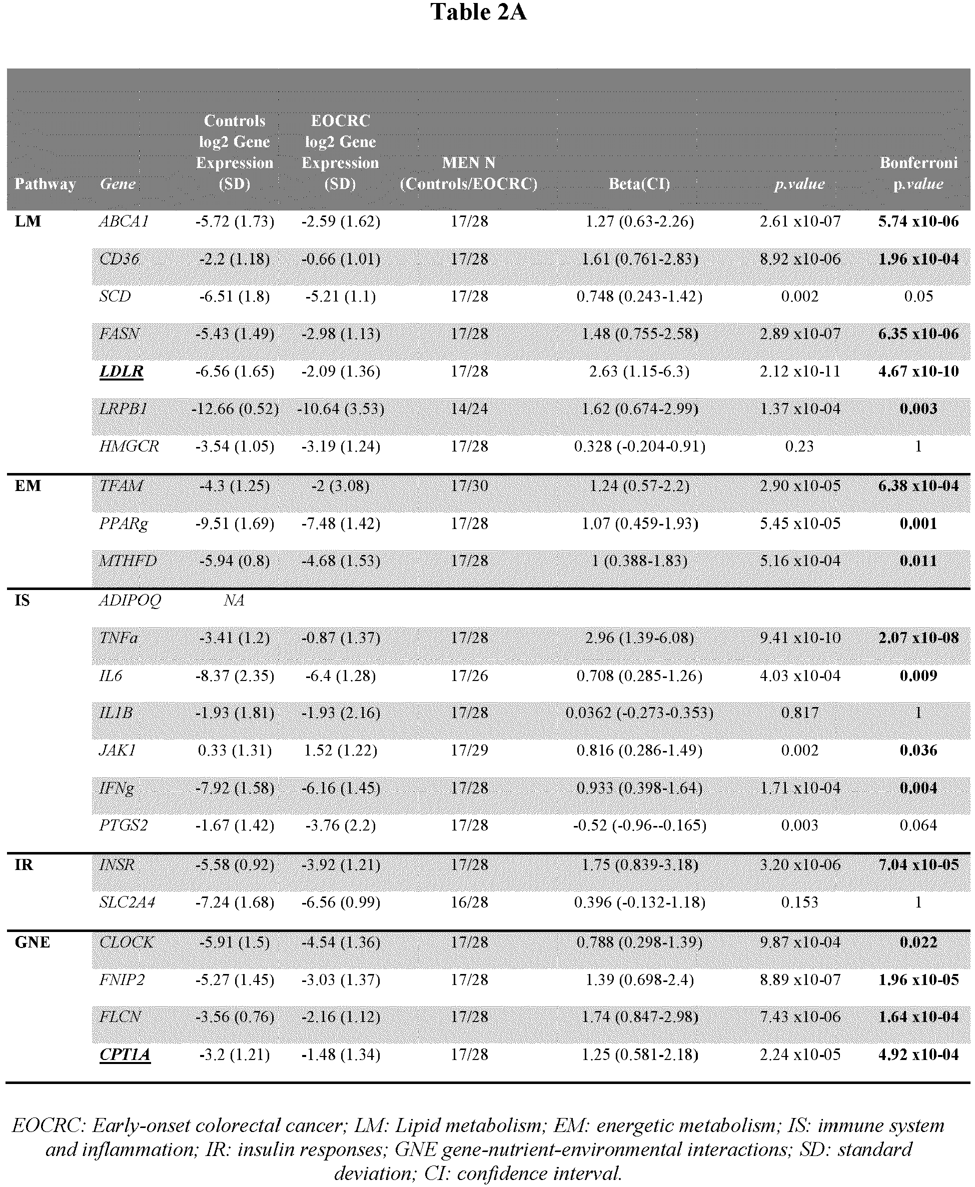

Conversely, the expression of two genes was exclusively associated to EOCRC in women: Prostaglandin-endoperoxide synthase 2 (*PTGS2,* also known as *COX2*)*,* that belongs to IS pathway (*beta*=-1.11 95%CI (-2.28, -0.452), *Bonferroni p-value*=0.001) and glucose transporter *SLC2A4* also known as *GLUT4* (IR pathway) (*beta*=2.37 95%CI (0.687, 5.16), *Bonferroni p-value*=0.01). Interestingly, *PTGS2* is the unique significant gene whose expression is downregulated among cases **(Table 2B).**

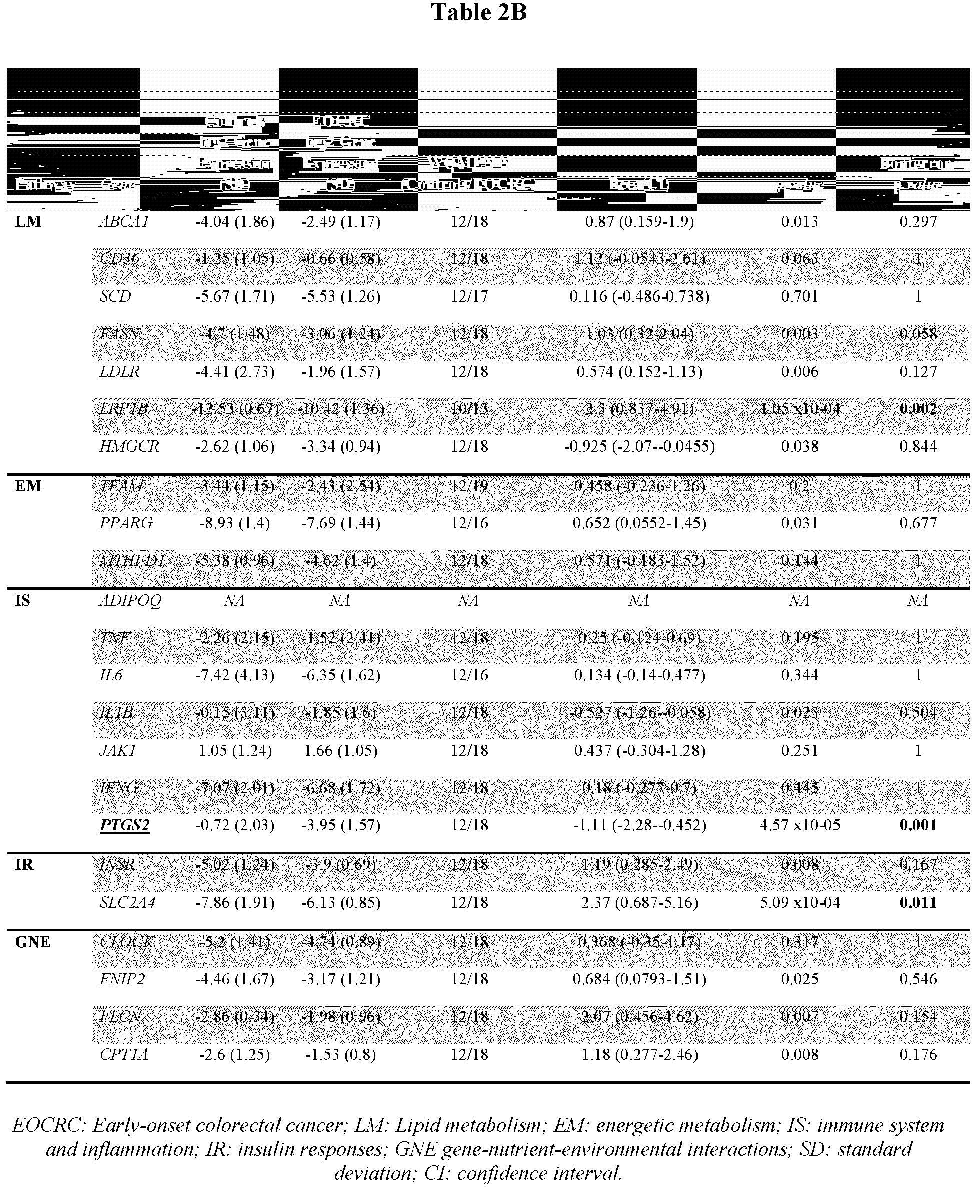

Only one gene, low-density lipoprotein receptor-related protein 1B (*LRP1B*) was upregulated in both men and women (*beta*=1.62 95%CI (0.674, 2.99), *Bonferroni p-value*=0.003) and *beta*=2.3 95%CI (0.837, 4.91), *Bonferroni p-value*=0.002) respectively*)*. *LRP1B* belong to the LM pathway. However, associated risk was slightly more increased among women indicating different effects or functions according to gender.

To find novel biomarkers of EOCRC, and potentially different according to sex, metabolic genetic risk scores (GRS) based on gene expression were developed. The models were validated through bootstrap resampling (500 resamples) and using the optimism-corrected c-index as measure of predictive capacity. EOCRC was associated with an upregulation of all metabolic health related pathways in men patients and among them, a GRS considering 2 genes: *LDLR* and *CPT1A* predicted CRC development with a *c-index* of 0.991. By contrast, women that developed EOCRC characteristically exhibited upregulation of IR routes and downregulation of immune responses. In women, only *PTGS2* expression was sufficient to predict EOCRC development (*beta*=-1.35 95%CI (-3.13, -0.463), *c-index*=0.896)*.*

In summary, the present invention disclosed clearly distinct metabolic scenarios according to sex influencing EOCRC development. While EOCRC in men is characterized with a total deregulation of metabolic landscape, EOCRC in women is focused on immune system and insulin responses routes **(****Figure 1****).** Altogether, the results provided in the present invention allow to design efficient personalized prevention for EOCRC patients, that should diminish disparities and EOCRC incidence.

## Claims

1. *In vitro* method for screening, diagnosis and/or prognosis of colorectal cancer in a subject which comprises:
a. Assessing the level of expression of the gene *PTGS2* in a biological sample obtained from the subject when the subject is a woman, in combination with the level of expression of the gene *LRP1B*, wherein the identification of a level of expression of the gene *LRP1B* higher, and a level of expression of the gene *PTGS2* lower, than a pre-established level of expression determined in healthy subjects, is an indication that the woman subject is suffering from colorectal cancer; or
b. Assessing the level of expression of the gene *LDLR* in a biological sample obtained from the subject when the subject is a man, in combination with the level of expression of the gene *LRP1B*, wherein the identification of a level of expression of the genes higher than a pre-established level of expression determined in healthy subjects, is an indication that the man subject is suffering from colorectal cancer.

2. *In vitro* method, according to claim 1, for screening, diagnosis and/or prognosis early-onset colorectal cancer in subjects under 50 years of age.

3. *In vitro* method, according to any of the previous claims, which comprises assessing the level of expression of the gene *LDLR* in combination with the level of expression of the genes *LRP1B* and *CPT1A* when the subject is a man.

4. *In vitro* method, according to any of the previous claims, wherein the biological sample is selected from serum, plasma or blood.

5. *In vitro* method, according to any of the previous claims, wherein the results are confirmed by means of an image technique, preferably colonoscopy.

6. *In vitro* use of the gene *PTGS2* in combination with the gene *LRP1B* for the screening, diagnosis and/or prognosis of colorectal cancer in women, or gene *LDLR* in combination with the gene *LRP1B* for the screening, diagnosis and/or prognosis of colorectal cancer in men.

7. *In vitro* use, according to claim 6, of the genes *LDLR* and *CPT1A* in combination with the gene *LRP1B* for the screening, diagnosis and/or prognosis of colorectal cancer in men.

8. *In vitro* use, according to any of the claims 6 or 7, for screening, diagnosis and/or prognosis early-onset colorectal cancer in subjects under 50 years of age.

9. Use of a kit consisting of reagents for assessing the level of expression of the gene *PTGS2* in combination with *LRP1B* in the screening, diagnosis and/or prognosis of colorectal cancer in women, or of a kit consisting of reagents for assessing the level of expression the gene *LDLR* in combination with *LRP1B* in the screening, diagnosis and/or prognosis of colorectal cancer in men.

10. Use, according to claim 9, of a kit consisting of reagents for assessing the level of expression the genes *LDLR* and *CPT1A* in combination with *LRP1B* in the screening, diagnosis and/or prognosis of colorectal cancer in men.

11. Use the kit, according to any of the claims 9 or 10, for screening, diagnosis and/or prognosis early-onset colorectal cancer in subjects under 50 years of age.

12. Kit consisting of reagents for assessing the level of expression of the gene *PTGS2* in combination with *LRP1B* or for assessing the level of expression the gene *LDLR* in combination with *LRP1B* and optionally with *CPT1A.*

13. *In vitro* method for recommending a treatment to a subject which comprises performing the method of any of the claims 1 to 5, wherein:
a. Treatment with inflammatory and immune system modulating agents is recommended to women subjects when a level of expression of the gene PTGS2 lower, and a level of expression of the gene LRP1B higher, than a pre-established level of expression determined in healthy subjects, have been identified;
b. Treatment with lipid-lowering agents and/or agents modulating lipid metabolism such as metformin or statins is recommended to men subjects when level of expression of the genes LDLR and/or CPT1A higher and level of expression of the gene LRP1B also higher, than a pre-established level of expression determined in healthy subjects, have been identified; or
c. Treatment with lipid-lowering agents and/or agents modulating lipid metabolism such as metformin or statins is recommended to woman subjects when level of expression of the gene LRP1B is higher than a pre-established level of expression determined in healthy subjects, have been identified.
